Europäisches Patentamt

European Patent Office

Office européen des brevets

(19)

(11) Publication number: **0 061 793**
**B1**

(12) **EUROPEAN PATENT SPECIFICATION**

(45) Date of publication of patent specification: **21.08.85**

(21) Application number: **82200281.2**

(22) Date of filing: **05.03.82**

(51) Int. Cl.⁴: **G 01 N 33/90, G 01 N 33/84**

(54) Method for preparing a chromogen reactive for determining the serum iron concentration and binding power, and the chromogen reactive obtained thereby.

(30) Priority: **01.04.81 IT 2085981**

(43) Date of publication of application:
**06.10.82 Bulletin 82/40**

(45) Publication of the grant of the patent:
**21.08.85 Bulletin 85/34**

(84) Designated Contracting States:
**AT BE CH DE FR GB LI NL SE**

(56) References cited:

**CLINICA CHIMICA ACTA, vol. 94, 1979
Amsterdam A. GARCIC "A Highly Sensitive,
Simple Determination of Serum Iron Using
Chromazurol B" pages 115 to 119
CLINICA CHIMICA ACTA, Vol. 114, 10 August
1981, Amsterdam A. TABACCO et al. "An
Improved, Highly Sensitive Method for the
Determination of Serum Iron Using
Chromazurol B" pages 287 to 290**

The file contains technical information
submitted after the application was filed and
not included in this specification

(73) Proprietor: **Brega, Augusta**
**Viale Europa, 72/G**
**I-25100 Brescia (IT)**

(72) Inventor: **Brega, Augusta**
**Viale Europa, 72/G**
**I-25100 Brescia (IT)**

(74) Representative: **Cicogna, Franco**
**Ufficio Internazionale Brevetti Dott.Prof. Franco
Cicogna Via Visconti di Modrone, 14/A
I-20122 Milano (IT)**

Courier Press, Leamington Spa, England.

## Description

The present invention relates to a method for making a homogeneous reagent for determining the iron contents and the iron binding capacity (TIBC) in serum, to an analytical method for determining the iron contents and iron binding capacity in serum (TIBC) and to the thus prepared reagent.

The serum contents of iron and transferrin (that is the iron contents upon saturation, or the total iron binding capacity=TIBC) may be determined by atomic absorption photometry or colorimetry, by using various chromogens, mainly those obtained from 1,10 phenantroline.

The determination of the iron contents by atomic absorption has not found a great development in the clinical-chemical field, both due to the high costs of the related apparatus and the difficulties relating to the use of said apparatus.

On the other hand, the colorimetry method is a very difficult procedure due to the low sensibility of the presently used chromogens (from 7 to $30 \times 10^3$ l.mol.$^{-1}$cm$^{-1}$).

More specifically this procedure involves a high sample-reactive ratio, with either one or the other of the following consequences:

a) it is necessary to pretreat the serum in order to remove proteins by means of precipitation and dialysis.

b) it is necessary to produce a blank for each sample in order to subtract from the final colour the serum proper colour.

Also the C.I. mordant blue 1/43830 colouring agent, which forms with iron and cetrimide a ternary complex provided with a high molar absorption at 630 nm (A$_{630nm}$1.6×10 l mol$^{-1}$cm$^{-1}$), has not been used in such a way as to eliminate the necessity of employing said sample blank (A. Garcic, Chim. Chem. Acta *94*, 115 (1979)).

Moreover the reagent had a maximum stability of one month only which, considering the complexity associated to the preparing thereof, involved great difficulties in use.

Obviously all of the thereinabove cited drawbacks relating to the determination of the serum iron contents or concentration are also found in the determination of transferring, (dosed as total iron binding capacity or power), since the two methods employ the common step of proportioning iron into the biological sample which latter, in the transferrin case, has been pretreated, as it will be illustrated in a more detailed way thereinafter.

Accordingly, the task of the present invention is that of providing a C.I. mordant blue 1/43830 containing chromogenic reagent which is effective to be broadly used in the clinical field, owing to the following main features:

— an easy application to the iron analysis in biological liquids, which is carried out without deproteinizing and without using blanks for each single sample,

— a stability of the liquid reagent of at least 6 months, susceptible to be increased as the reagent is prepared in the granulated state and then solubilized with a suitable buffer at the time of use,

— an easy application for determining the transferrin contents, as iron binding power, since in the end stage of the analytical procedure it is used for proportioning the carrier protein bonded iron.

All of the mentioned features provide a reagent effective to be easily used on the several types of automatic apparatus existing in the clinical analysis laboratories.

Moreover they afford the possibility of simultaneously carrying out on those same apparatus the iron analysis and the final stage of the serum transferrin (TIBC) analysis.

According to one aspect of the present invention, the thereinabove mentioned task is achieved by a method for preparing a chromogenic reagent, characterized in that it comprises the steps of preparing the free acid C.I mordant blue 1/43830, preparing an ammonium salified mordant blue 1/43830 based powder, preparing buffers for determining the serum iron contents and iron binding capacity, and preparing a liquid reagent by adding from 0.8 to 3 g of granulate to 125 ml of buffer and then dissolving.

Further characteristics and advantages will become more apparent hereinafter from the following detailed description, with reference to the accompanying drawings, where:

Fig. 1 illustrates by experimental data the linearity of the instant method for determining the serum iron contents or concentration up to 500 ml/dl;

Fig. 2 illustrates the absorbancy spectrum.

The method for preparing the chromogenic reagent and the analytical method, both for the serum iron and the total iron binding power, will be described in a detailed way hereinafter with reference to the results of the carried out experiments.

Preparing of the chromogenic reagent in the liquid and granulated state

1) Purifying of the C.I mordant blue 1/43830 in free acid form: dissolve in about 250 ml water from 2 to 10 g (for example 4 g) of the commercially available product and mix with an equal volume of about 1 mole/1 HCl. Decant and filter. Repeat the cited operations several times, i.e. five times. Dry at 95°C and mill to a fine powder state or lyophilize.

2) Preparing of the ammonium salified mordant blue 1/43830 based powder:weigh from 100 to 200 mg

# 0 061 793

(for example 150 mg) of purified mordant blue and about 37g of NaCl; homogenize by milling in a mill; treat with anhydrous ammonia or ammonia salts (for example commercially available ammonia), until the powder assumes a deep brown colour; introduce accurate amounts from 0.8 to 3 g (for example 1.5 g) into vials and tightly seal.

3) Preparing of the buffers for determining the serum iron contents or concentration and iron binding power; dissolve in a 1 litre flask, in about 800 ml of bi-distilled water, about 25 g of anhydrous sodium acetate and about 100 g NaCl and bring to a pH varying from 4.5 to 5 (for example 4.7) with glacial acetic acid; add from 0.2 to 0.5 (for example 0.47) g of cetylmethylammoniumbromide. Bring to volume. Distribute 125 ml of buffer for each vial.

4) Preparing of the chromogen reactive:

In order to prepare the liquid reactive, from 0.8 to 3 g of granulate (for example 1.5 g) are added to 125 ml of buffer and the mixture is dissolved.

The bivalent iron reacts with C.I mordant blue 1/43830 and cetyltrimethylammoniumbromide in such a way as to form, at ambient temperature, a high molar extinction coefficient ternary complex; the colour intensity, read at 630 nm (620—640 nm) against the blank is proportional to the iron concentration up to 80 μmol/l. The serum iron binding capacity or power, expressed in μg of iron/dl, is determined by saturating the transferrin with iron in the form of a concentration solution, removing the excess iron by means of absorption on magnesium carbonate powder and subsequent centrifugation and proportioning the iron present in the overlying or surnageant layer by means of the same method used for determining the iron concentration.

The analytical methods hereinbelow illustrated represent two possible indicative examples. Obviously the proportions of the samples with respect to the reagents have to be met.

Determination of the serum iron

Pipette into serupulously clean test tubes 50 μl of water (blank), of standard and of sample. Add 1.2 ml of chromogenic reagent to each tube. Stir, waiting for 10 minutes and read out at 630 nm the absorbancies of the samples and of the standard against the blank.

The concentration of iron in the serum sample is calculated based on the following formula:

$$\text{serum iron, μg/dl} = \frac{A\ \text{sample}}{A\ \text{standard}} \times \text{the standard value}$$

Determination of the total serum capacity (TIBC):

Pipette into a reduced diameter test tube 0.5 ml serum, add 10 μl of saturating agent, mix and wait for ten minutes.

Add a spatula tip of basic magnesium carbonate powder, mix or stir each 5 minutes for three times on V rtex. Centrifuge at high speed for ten minutes. Treat as indicated for the iron determination:

$$\text{transferrin (TIBC)μg/dl} = \frac{A\ \text{sample}}{A\ \text{standard}} \times \text{value of the standard}$$

Results:

a) Colour development

By using a serum/chromogenic reagent ratio of 1:25, the formation rate of the coloured complex is greater in the first two minutes and it reaches a maximum in 5 min.

The colour is stable for over five hours.

b) Blank absorbancy

The blank absorbancy or absorption power of the samples (50 μl serum+1.2 ml of buffer-cetrimide mixture), read out against water at 630 nm were of 0.003±0.001 (n=100; range from 0.001 to 0.008).

It should be noted that the blank absorbancy of control commercially available sera, for example the Kontrollogen L of the Istituto Berhing, have very high values.

c) Mordant blue concentration

The concentration of the mordant blue is not critical and it can vary from 30 to 150 mg/litre.

d) Linearity and absorption spectrum

The proposed method is linear up to 500 μg/dl (Fig. 1). The absorption spectrum has its maximum at 630 nm (Fig. 2).

e) Stability of the reagent

There have been recorded monthly, for six consecutive months, the absorption powers or absorbancies of the chromogenic reagent, measured against water, and the absorption powers of the

3

**0 061 793**

reaction mixtures with the standard and a control serum, measured against a blank. The data indicated in the following Table 1 indicate a progressive increasing of the absorption power of the reagent which, however, does not affect the results, expressed as concentration.

TABLE 1

Stability of the chromogenic reagent

| Months | Absorbancy at 630 nm | | Sample concentration (µg/dl) |
| | chromogenic reagent | standard | |
| --- | --- | --- | --- |
| 0 | 0.391 | 0.171 | 0.141 |
| 1 | 0.390 | 0.181 | 0.142 |
| 2 | 0.394 | 0.180 | 0.143 |
| 3 | 0.399 | 0.191 | 0.141 |
| 4 | 0.411 | 0.179 | 0.143 |
| 5 | 0.420 | 0.184 | 0.144 |
| 6 | 0.427 | 0.182 | 0.141 |

f) Correlation with other methods

Parallel experiments with the known Martinek method, modified for iron, and moreover with the Lauber methods and the radial immunodiffusion technique according to Fahey and McKelvy for transferrine (TIBC) (by using M plates—Partigen Istituto Behring) have provided the data illustrated in Table 2.

TABLE 2

Correlation of the iron and transferrin results obtained by several methods comparable with the method according to the present invention

| Method | Iron | | | | Transferrin | | | |
| | n | r | a | b | n | r | a | b |
| --- | --- | --- | --- | --- | --- | --- | --- | --- |
| Mod. Martinek | 87 | 0.9674 | 8.2 | 1.0 | 25 | 0.9348 | 32.8 | 0.9 |
| Rad. Immunodiff. | — | — | — | — | 25 | 0.9269 | 57.8 | 0.6 |
| Lauber | — | — | — | — | 25 | 0.9863 | 24.8 | 1.0 |

where

r=correlation coefficient
a=intercept
b=angular parameter.

g) Reproducibility

The results of the determinations carried out on a serum set containing an average iron and transferrin contents and on the standard product are indicated in the following Table 3.

4

## TABLE 3
### Reproducibility of the determinations

|  | n | µg/dl | ±s | Range | CV% |
|---|---|---|---|---|---|
| **within the series** |  |  |  |  |  |
| Iron pool | 20 | 89 | 1.7 | 85—91 | 1.9 |
| Standard | 30 | 181 | 2.9 | 176—185 | 1.6 |
| Transferrin pool | 20 | 376 | 12 | 360—408 | 3.2 |
| **between the series** |  |  |  |  |  |
| Iron pool | 22 | 72 | 3.1 | 65—76 | 4.3 |
| Transferrin pool | 20 | 354 | 28 | 330—391 | 7.9 |

h) Accuracy

The accuracy of the method has been evaluated by analyzing commercially available control sera, of known value (Table 4). Some sera have not been found to be suitable since they rendered the reaction mixture opaque (for example Kontrollegen L-Ist. Behring) or since they contained interfering preserving substances (for example Precinorm U—Biochemia; Tek chek—Miles).

## TABLE 4
### Accuracy control

| Serum | From the company | Theoretical value µg/dl | Experimental value µg/dl |
|---|---|---|---|
| **Iron** |  |  |  |
| Control serum N | Roche | 138 | 135 |
| Control serum A | " | 171 | 168 |
| Seronorm | Nyegaard | 121 | 109 |
| SVRS | Wellcome | 111 | 112 |
| **Transferrin** |  |  |  |
| Seronorm | Nyegaard | 420 | 390 |
| Contro serum N | Roche | 387 | 381 |

i) Reference intervals

The data obtained both for iron and transferrin (total iron binding capacity) are reported in Table 5.

## TABLE 5
### Reference intervals

|  | n | µg/dl | s | Range |
|---|---|---|---|---|
| Iron: o | 100 | 127 | 29 | 65—200 |
| o | 100 | 109 | 29 | 50—188 |
| transferrin | 25 | 332 | 36 | 270—390 |

From the above description it should be noted that the invention has several main features, among which the following are specifically cited:

# 0 061 793

1) The stability of at least six months of the liquid reagent according to the present invention for determining the iron contents or concentration in the biological liquids and the transferrin contents in serum, which reagent comprises mordant blue 1/43830, ammonium salts and cetylammoniumbromide, in an acetate buffer at a pH of 4.7.

The originality of the instant reagent is to be pointed out, in addition to the fact that the present invention does not consist of the use of the chromogenic C.I mordant blue 1/43830, but, on the contrary, of the reagent composition which imparts thereto the stability which is essential for its diffusion in the clinical practice.

2) The preparation of that same reagent in the granulate state by means of an effectively new method.

In this connection it should be pointed out that the invention consists of the specific steps thereby there is carried out the drying process, since it is not possible to directly dry the reagent in the liquid state.

3) The analytical method by means of the instant reagent for determining the serum iron concentration has the following new characteristics:

a) the need is obviated of carrying out any deproteinizing steps;

b) the need is also obviated of using any sample blanks.

4) The analytical method by means of the instant reagent may also be used for determining the serum transferrin contents or concentration.

Accordingly the new characteristics of the present invention consist both of the preparing of a powder in which the coloring agent remains salified in a stable form, and of the analytical method which does not require the preparing of the sample blank, and, finally, of the extension of the method to the determination of the serum iron binding capacity (TIBC) or power.

**Claims**

1. A method for preparing a chromogenic reagent for determining the iron contents and the iron binding capacity in serum, said chromogenic reagent comprising the ammonium salt of C.I. mordant blue I/43830, together with a buffer solution having a pH of 4.5—5 containing cetyltrimethylammonium bromide, characterized in that the method consists in the following steps, the weights and volumes given in the preparation and mixing steps being exemplary and definitive only of the relative proportions of the components, similarly the quantities in the introduction step and the distribution step are definitive only of the relative proportion of powder to buffer solution:

— preparing the free acid form of the C.I. mordant blue I/43830 and milling to a powder state said free acid;
— mixing 100 to 200 mg of the above free acid form of the C.I. mordant blue I/43830 with about 37 g of NaCl and homogenizing the mixed powder;
— treating the resulting mixture with anhydrous ammonia or an ammonium salt until the salified powder assumes a deep brown color;
— introducing accurate amounts from 0.8 to 3 g of the above salified powder into first vials which are then tightly sealed;
— preparing the buffer solution by dissolving 25 g of sodium acetate and 100 g of NaCl in 800 ml of water, bringing the solution to a pH of 4.5—5 with glacial acetic acid, adding from 0.2 to 0.5 g cetyltrimethylammonium bromide and bringing to 1 l volume; and
— distributing 125 ml of the above buffer solution into each of second vials which are then sealed.

2. A chromogenic reagent obtained by dissolving 0.8 to 3 g of the salified powder of claim 1 in 125 ml of the buffer solution of claim 1.

3. A method for determining serum iron concentration, characterized in that it consists in pipetting into respective test tubes 50 µl each of water (blank), of standard and of sample, together with 1.2 ml of the chromogenic reagent of claim 2, stirring the solutions and after ten minutes reading at 630 nm the absorbencies (A) of the standard and the sample against the blank, and calculating the serum iron contents by the formula:

$$\text{serum iron concentration (µg/dl)} = \frac{\text{A sample}}{\text{A standard}} \times \text{serum iron concentration of standard}$$

4. A method for determining the serum transferrin concentration, characterized in that it consists in pipetting into a test tube 0.5 ml of serum, adding 10 µl of a saturating agent, stirring the obtained mixture, waiting for ten minutes, adding a small amount of basic magnesium carbonate powder, stirring each 5 min. for three times, centrifuging for 10 min. at high speed, treating 50 µl of surnatant with 1.2 ml of the chromogenic reagent of claim 2, stirring the solution and after ten minutes reading at 630 nm the absorbencies (A) of the sample, and calculating the total serum capacity by the formula:

$$\text{total serum capacity (µl/dl)} = \frac{\text{A sample}}{\text{A standard}} \times \text{total serum capacity of standard}$$

6

# 0 061 793

Patentansprüche

1. Verfahren zur Herstellung eines chromogenischen Reaktandes zur Bestimmung des Inhalt an Eisen und der Bindungsfähigkeit des Eisens in einem Serum, wobei der chromogenische Reaktand das Ammoniumsalz von C.I mordant blue I/43830 zusammen mit einer Äthyltrimethylammonium Bromid enthaltenden Pufferlösung mit einem pH-Wert von 4,5 bis 5 enthält, dadurch gekennzeichnet, dass das Verfahren in den folgenden Stufen besteht, wobei die während der Vorbereitungs- und Mischungsstufen gegebenen Gewichte und Volumen, welche nur die jeweiligen Verhältnisse der Bestandteile bestimmen, ähnicherweise die Mengen in der Zumischungsstufe und in der Verteilungsstufe, die nur die jeweiligen Verhältnisse von Pulver und Pufferlösung angeben, nur Beispiele sind:

— Vorbereitung der Form von freier Säure des C.I. mordant blue I/43830 sowie Vermahlung zu einem Pulverzustand der freien Säure;
— Zumischung von 100 bis 200 mg der Form von vorhergehender freier Säure des C.I. mordant blue I/43830 mit etwa 37 g NaCl sowie Homogenierung des gemischten Pulvers;
— Behandlung der entstehenen Mischung mit wasserfreiem Ammoniak oder mit einem Ammoniumsalz, bis das gesalzte Pulver eine dunkel-braune Farbe eingenommen hat;
— Einbringung von bestimmten Mengen von 0,8 bis 3 g des vorhergehenden gesalzten Pulvers in erste, dicht versiegelte Phiolen;
— Vorbereitung der Pufferlösung, indem man 25 g Natriumazetat und 100 g NaCl in 800 ml Wasser löst, die Lösung zu einem pH-Wert von 4,5 bis 5 mit eisiger Essigsäure führt, 0,2 bis 0,5 g Kethyltrimethylammonium Bromid zumischt und zu Volumen bringt; und
— Verteilung von 125 ml der vorhergehenden Lösung in jede von zweiten Phiolen, die danach gesiegelt werden.

2. Chromogenisches Reaktand, das erhalten wird, indem man 0,8 bis 3 g des gesalzten Pulvers nach Anspruch 1 in 125 ml der Pufferlösung des Anspruches 1 löst.

3. Verfahren zur Bestimmung des Konzentration von Eisen- Ionen, dadurch gekennzeichnet, dass man in jeweiligen Probiergläser je 50 microliter Wasser (Weiss) von Standard und Probe zusammen mit 1,2 ml des chromogenischen Reaktandes nach Anspruch 2 durch Stechheber einspritzt, die Lösung mischt und nach 10 Minuten die Adsorptionsvermögen (A) des Standards und der Probe in Bezug auf das Weiss liest, und den Inhalt an Eisen im Serum durch die Formel:

$$\text{Eisenkonzentration im Serum (microgramm/dl)} = \frac{A \text{ probe}}{A \text{ standard}} \times \text{Eisenkonzentration im Serum des standards}$$

rechnet.

4. Verfahren zur Bestimmung der Konzentration des Transeisen-Serums, dadurch gekennzeichnet, dass in ein Probierglas 0,5 ml Serum durch Stechheber eingespritzt werden, 10 Microliter eines sättigenden Mittels zugelegt werden, die erhaltene Lösung gerührt wird, nach 10 Minuten Warten eine kleine Menge an Pulver von basischem Magnesiumkarbonat zugelegt wird, je 5 Minuten drei Male gerührt wird, 10 Minuten lang mit hoher Geschwindigkeit zentrifugiert wird, 50 Microliter Schwemmsel mit 1,2 ml des chromogenischen Reaktandes, nach Anspruch 2 behandelt werden, die Lösung gerührt wird und nach 10 Minuten bei 630 nm die Adsorptionskoeffizienten (A) der Probe gelesen werden und das gesamte Vermögen des Serums durch die Formel:

$$\text{gesammtes Vermögen des Serums (Microliter/dl} = \frac{A \text{ probe}}{A \text{ standard}} \times \text{gesamtes Vermögen des Serums des Standards}$$

gerechnet wird.

Revendications

1. Méthode pour la préparation d'un réactif chromogénique pour déterminer le contenu en fer et la capacité de liason du fer dans sérum, ledit réactif chromogénique comprenant le sel d'ammonium de C.I. mordant blue I/43830 avec une solution tampon ayant un pH comprise entre 4,5 et 5 contenante éthyltriméthylammonium bromure, caractérisée en ce que ladite méthode consiste dans les étages suivants, les poids et les volumes donnés dans l'étage de préparation et de mélange étant donnés à titre d'exemple et définissant seulement les proportions rélatives des composants, d'une manière analogue à les quantités dans l'étage d'introduction et dans l'étage de distribution, lesquelles sont indicatives seulement des proportions rélatives de poudre et de solution tampon:

— préparation de la forme d'acide libre du C.I. mordant blue I/43830 et broyage dans l'état de poudre dudit acide libre;

— mélange de 100—200 mg of the forme d'acide libre ci-dessus mentionnée du C.I. mordant blue I/43830 avec environ 37 g de NaCl et homogénéization de la poudre mélangée;

— traitement du mélange obtenu avec ammoniaque anhydre ou avec un sel d'ammonium jusqu'à l'obtention par la poudre salifiée d'une coloration marrone foncée;

— introduction de quantités précises de 0,8—3 g de ladite poudre salifiée dans des premières ampoules les-quelles sont scellées d'une manière étanche;

— préparation de la solution tampon en dissolvant 25 g d'acétate de sodium et 100 g de NaCl dans 800 ml d'eau, en portant la solution à un pH de 4,5—5 avec acide acétique glaciale, en additionnant 0,2—0,5 g de cétyltriméthylammonium bromure et portant à volume; et

— distribution de 125 ml de la solution tampon ci-dessus mentionnée dans chaque de deuxième ampoules lesquelles sont succéssivement scellées.

2. Réactif chromogénique obtenu en dissolvant de 0,8 à 3 g de la poudre salifiée selon la revendication 1 dans 125 ml de la solution tampon selon la revendication 1.

3. Méthode pour déterminer la concentration d'ions fer, caractérisée en ce que ladite méthode consiste dans pipetter dans des ampoules respectives 50 microlitres pour chaqune d'elles d'eau(blanc) de standard et d'échantillon, avec 1,2 ml du réactif chromogénique selon la revendication 2, agiter les solutions et, après 10 minutes, lire à 630 nm la capacité d'absorption (A) du standard et d'échentillon par rapport au blanc, et calculer le contenu de fer dans le sérum par la formule:

$$\text{concentration de fer dans le sérum (µg/dl)} = \frac{\text{A échantillon}}{\text{A standard}} \times \text{concentration de fer dans le sérum du standard}$$

4. Méthode pour déterminer la concentration de sérum-transferrine, caractérisée en ce que ladite méthode consiste dans pipetter dans une ampoule 0,5 ml de sérum, additionner 10 microlitres d'un agent saturant, agiter le mélange obtenu, attendre pour 10 minutes, additionner une petite quantité de poudre de carbonate de magnésium basique, agiter tous le 5 minutes, pour trois fois, centrifuger pour 10 minutes avec une vitesse haute, traiter 50 microlitres de surnageant avec 1,2 ml du réactif chromogénique selon la revendication 2, agiter la solution et lire, après 10 minutes, à 630 nm la capacité d'absorption (A) de l'échantillon, et calculer la capacité totale du sérum per la formule:

$$\text{capacité totalt du sérum (µl/dl)} = \frac{\text{A échantillon}}{\text{A standard}} \times \text{capacité totale du sérum du standard}$$